# FASCICULE DE BREVET EUROPEEN

(11) **EP 2 393 521 B1**
(45) Date de publication et mention de la délivrance du brevet: **12.09.2018**
(21) Numéro de dépôt: 10707081.5
(22) Date de dépôt: 09.02.2010
(51) Int. Cl.: A61L 2/00, A61L 2/10, A61L 2/08

(54) **PROCÉDÉ POUR MODIFIER LES PROPRIÉTÉS D'UN FLUIDE PAR IRRADIATION ET SYSTÈME POUR SA MISE EN OEUVRE**
VERFAHREN ZUR ÄNDERUNG DER EIGENSCHAFTEN EINER FLÜSSIGKEIT MITTELS BESTRAHLUNG UND SYSTEM ZUR UMSETZUNG DIESES VERFAHRENS
METHOD FOR MODIFYING THE PROPERTIES OF A FLUID BY IRRADIATION, AND SYSTEM FOR IMPLEMENTING SAME

(30) Priorité: 09.02.2009 FR 0900562
(43) Date de publication de la demande: 14.12.2011
(73) Titulaire: Maco Pharma, 59400 Mouvaux (FR)
(72) Inventeur: BONTINCK, Pierre-Eloi, F-59800 Lille (FR)
(74) Mandataire: Sayettat, Julien Christian
(86) Numéro de dépôt international: PCT/FR2010/000098
(87) Numéro de publication internationale: WO 2010/089485

(56) Documents cités:
- WO-A-00/20045
- WO-A-02/38191
- WO-A-2004/037301
- WO-A-2007/137245
- US-B1- 6 951 548

## Description

L'invention concerne un procédé pour modifier les propriétés physiques, chimiques et/ou biologiques d'un fluide par irradiation, ainsi qu'un système pour la mise en oeuvre d'un tel procédé.

L'invention s'applique au domaine du traitement par irradiation des fluides tels que les fluides biologiques destinés à être utilisés en diagnostic, nutrition, thérapie, y compris la thérapie cellulaire. Notamment, l'invention s'applique à la réduction des pathogènes d'un fluide tel que le sang ou les composants sanguins à l'aide d'un rayonnement seul, par exemple ultraviolet-C, ou de l'association d'une substance photosensible avec un rayonnement, par exemple de la lumière visible, infrarouge ou ultraviolette.

On connaît du document WO 2007/076834 une méthode pour inactiver les pathogènes d'un concentré plaquettaire contenu dans un récipient par agitation et irradiation conjointe à l'aide d'ultraviolet. L'agitation permet une irradiation statistiquement uniforme du concentré plaquettaire.

Cependant, dans le cas de fluides très concentrés ou plus généralement qui absorbent fortement le rayonnement considéré, comme un concentré de globules rouges, cette méthode nécessiterait soit d'utiliser un récipient de taille très grande, soit d'augmenter considérablement la durée d'irradiation, rendant cette méthode impraticable en routine dans les centres de traitement du sang.

On connaît par exemple du document WO 2006/128047 un système d'irradiation du sang dans lequel le sang est exposé à une irradiation aux ultraviolets alors qu'il circule dans un tube en plastique.

Ce système en flux ne permet cependant pas d'obtenir une couche suffisamment mince pour permettre la pénétration de la lumière dans un fluide concentré.

Il est connu des documents WO2004/037301 A2, WO 00/20045, US 6 951 548 B1, WO 02/38191 A2 et WO 2007/137245 A2 différents systèmes et méthodes pour irradier des fluides afin de les stériliser.

Il existe des dispositifs micro-fluidiques notamment destinés au tri, à la séparation et/ou à la détection de particules suspendues dans un fluide. Un tel dispositif est par exemple décrit dans le document WO 2008/130977. Dans ce dispositif, les particules sont focalisées et ordonnées avant d'être séparées et analysées.

L'invention propose une méthode permettant de contrôler et/ou garantir la dose de rayonnement reçue par un fluide. Elle permet également d'irradier le fluide sur l'épaisseur, même dans le cas de fluides contenant des éléments opaques au rayonnement considéré.

A cet effet et selon un premier aspect, l'invention propose un procédé pour modifier les propriétés physiques, chimiques et/ou biologiques d'un fluide par irradiation avec une dose d'un rayonnement, tel que défini dans la revendication 1.

Selon un deuxième aspect, l'invention propose un système pour la mise en oeuvre d'un tel procédé, tel que défini dans la revendication 10. D'autres objets et avantages apparaîtront au cours de la description qui suit.
La figure 1 représente une vue schématique en éclatée d'une chambre d'irradiation selon une première réalisation de l'invention.
La figure 2 représente une vue schématique et en perspective d'une chambre d'irradiation avec ses périphériques.
La figure 3a représente de façon schématique, dans une coupe d'une chambre d'irradiation, près des entrées, les lignes du courant de trois fluides ayant des débits égaux.
La figure 3b représente de façon schématique, dans une coupe d'une chambre d'irradiation, près des entrées, les lignes du courant de trois fluides, lorsque le débit de l'entrée centrale est plus faible que les débits des entrées supérieure et inférieure.
La figure 3c représente de façon schématique, dans une coupe d'une chambre d'irradiation, près des entrées, les lignes de courants de trois fluides, lorsque le débit de l'entrée inférieure est plus élevé que les deux autres.
La figure 4 représente une vue schématique et en coupe d'une portion d'une chambre d'irradiation dans laquelle l'écoulement est laminaire et le profil des vitesses est parabolique.
La figure 5 représente une vue schématique en coupe d'une chambre d'irradiation pourvue de deux entrées et deux sorties.
La figure 6 représente une vue schématique d'une chambre d'irradiation à trois entrées et trois sorties ainsi qu'un dispositif d'illumination.
La figure 7 représente une vue schématique en coupe d'une chambre destinée au mélange de deux fluides.
La figure 8 représente une vue schématique en coupe d'une chambre destinée à la séparation de deux types de particules contenues dans un fluide par couplage avec une force de séparation, telle que la gravité, un champ magnétique, électrique, ultrasonore, la force centrifuge ou encore les forces de portances des particules près de la paroi.

L'invention concerne un procédé pour modifier les propriétés physiques, chimiques et/ou biologiques d'un fluide par irradiation contrôlée avec une dose d'un rayonnement.

Le fluide à traiter par irradiation est par exemple un fluide biologique tel que le sang total ou un composant sanguin. Les composants sanguins comprennent notamment les produits obtenus par aphérèse ou par centrifugation du sang total, tel que le plasma, les plasmas riches en plaquettes, les concentrés plaquettaires, les concentrés de globules, et les couches leuco-plaquettaires. En variante, le sang total ou les composants sanguins sont déleucocytés ou appauvris en leucocytes, par passage au travers d'un filtre à déleucocyter. L'irradiation inclut l'émission d'ondes électromagnétiques comprenant notamment les rayons X, les rayons gamma, les ondes infrarouges, visibles ou ultraviolettes.

L'irradiation est notamment réalisée pour réduire les contaminations pathogènes d'un fluide. Par exemple, lorsque le fluide est de l'eau, un traitement par irradiation aux ultraviolets C (UVC) permet d'éliminer ou de réduire le nombre de bactéries actives présentes dans le fluide.

Dans le cas où le fluide est un fluide biologique comme le sang ou un composant sanguin, il peut être additionné d'une substance photosensible telle qu'une phénothiazine (bleu de méthylène), un psoralène (méthoxypsoralène) ou un dérivé de riboflavine (vitamine B2). Une irradiation appropriée (lumière visible ou UV) du fluide additionné de la substance photosensible permet alors de réduire les pathogènes, notamment bactéries et/ou virus, présents dans le fluide.

En variante, le sang total ou le composant sanguin à irradier est additionné d'une solution additive et/ou de conservation. Par exemple, le concentré de globules rouges est additionné d'une solution additive de type SAGM (saline - adénine - glucose mannitol). Le concentré de plaquettes est additionné d'une solution de conservation de type SSP+ commercialisée par Macopharma.

En variante, l'irradiation seule, par exemple avec un rayonnement UV ayant une longueur d'onde de l'ordre de 254 nm d'un concentré plaquettaire ou d'un concentré de globules rouges est réalisée pour réduire les bactéries, les virus et les parasites actifs éventuellement présents, ainsi que les leucocytes.

Dans une autre variante, l'irradiation des plaquettes avec un rayonnement UVA est utilisée pour réduire leur immunogénicité chez un patient alloimmunisé.

En particulier dans le cas où le fluide à traiter est un fluide biologique, il est important d'appliquer une dose de rayonnement suffisante pour obtenir l'effet souhaité, par exemple, une décontamination des pathogènes, tout en minimisant les dommages occasionnés aux éléments du fluide.

Selon l'invention, le fluide est focalisé de sorte à créer un écoulement dont la géométrie, par exemple en formant une couche mince dont l'épaisseur est constante et déterminée, est agencée pour que la dose de rayonnement modifie les propriétés du fluide. La focalisation peut être réalisée par action d'un champ de force, notamment un champ hydrodynamique, permettant d'obtenir des couches minces micrométriques.

Ainsi, même les fluides concentrés tels que les concentrés de globules rouges peuvent être traités.

Le procédé selon l'invention permet d'obtenir de façon certaine une telle épaisseur constante de couche de fluide à irradier, sans nécessiter un récipient ayant une épaisseur aussi faible que la couche.

Selon l'invention, le procédé comprend l'amenée d'un écoulement du fluide à irradier dans une chambre d'irradiation puis l'application d'une dose de rayonnement sur l'écoulement du fluide dans une portion déterminée de la chambre d'irradiation. L'écoulement est contrôlé, c'est-à-dire que son débit est fixé.

La chambre d'irradiation est au moins partiellement perméable au rayonnement à appliquer, c'est-à-dire que l'intensité d'au moins une partie du rayonnement incident est transmise à l'intérieur de la chambre d'irradiation.

La totalité ou une partie seulement de la chambre est perméable au rayonnement à appliquer.

Par exemple, dans le cas d'un rayonnement électromagnétique, la chambre ou la partie de chambre perméable au rayonnement est réalisée en polycarbonate, quartz, verre, polystyrène, acrylique, polychlorure de vinyle ou polyméthyl méthacrylate.

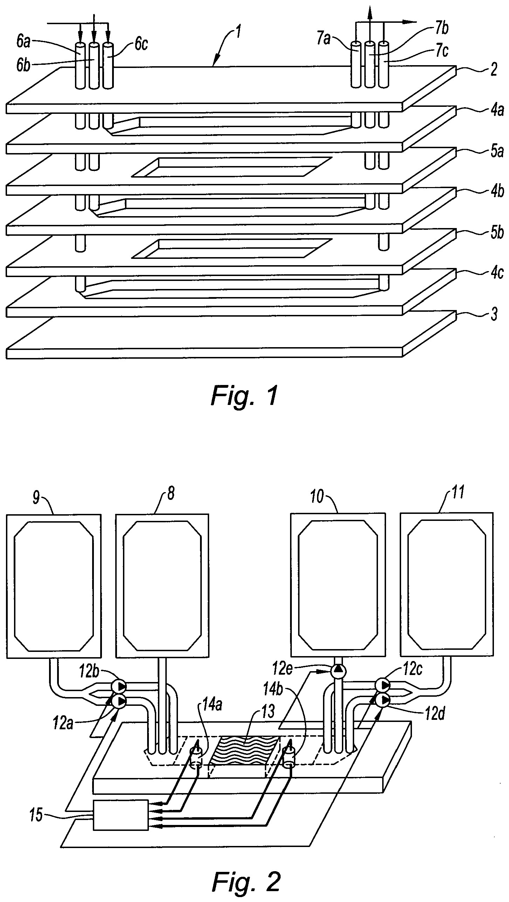

La chambre est généralement formée par assemblage de plaques fines de géométries (forme et dimensions) variables. Une réalisation particulière de la chambre d'irradiation est illustrée sur la figure 1. Selon cette figure, la chambre 1 est constituée de plaques fines rectangulaires et parallèles entre elles, telle que des films 2;3,4a,4b,4c,5a,5b de polymères et/ou ou de métaux. Les films 2,3 intervenants dans la transmission du rayonnement lui sont perméables. En particulier, la largeur de la plaque est comprise entre 1 et 50 cm et la longueur entre 5 et 100 cm.

Les plaques définissent une voie d'écoulement sous forme d'un canal d'épaisseur micrométrique ou millimétrique dans lequel la portion de la chambre d'irradiation est formée. En particulier, l'épaisseur du canal est comprise entre 0,1 mm et 5 cm et plus particulièrement entre 0,5 mm et 0,5 cm.

Par exemple, deux plaques parallèles d'environ 3 cm par 15 cm sont séparées d'une distance d'environ 1 mm.

Cette chambre particulière possède une symétrie planaire caractérisée par le plan passant au centre de la chambre, orthogonal à la surface des plaques la constituant et au canal.

La chambre 1 possède une épaisseur suffisamment faible pour que les écoulements à l'intérieur de celle-ci soient laminaires.

Un écoulement laminaire est caractérisé par un nombre Reynolds faible, notamment inférieur à 2320. Le nombre Reynolds est définit par l'équation Re=(ρVd/µ) dans laquelle p est la densité du fluide, V est la vélocité du fluide, d est la dimension de l'écoulement et µ est la viscosité du fluide.

Le cas échéant, la superposition de couches de fluide dans la chambre est largement hétérogène. Si on considère le cas le plus sévère, le nombre de Reynolds le plus grand que l'on puisse obtenir est lié à une densité, une vitesse et une dimension importantes, ainsi qu'à une viscosité faible. Un grand nombre de Reynolds entraîne un régime de fonctionnement moins laminaire.

Par exemple, pour un concentré de globules rouges à 70% d'hématocrite entrant dans une chambre entre deux couches de chlorure de sodium à 0.9%, la densité maximale pour les globules rouges de l'ordre de 1.08 g/cm³ et la viscosité minimale de l'ordre de 1 Pa.s pour le chlorure de sodium. Avec des hypothèses géométriques telles que l'épaisseur du canal de la chambre égale à 1 mm, et une vitesse maximale à 3,3 cm/s au centre de la chambre, le nombre de Reynolds calculé est de l'ordre de 0,032 ; soit un fonctionnement très laminaire.

Avantageusement, le ratio largeur/épaisseur est important pour que les effets de bords soient faibles ou négligeables.

Selon une variante, un support rigide plus épais qui n'est pas représenté dans la figure 1 est utilisé pour conformer la chambre et compenser la souplesse éventuelle des films utilisés. Ce support évite la déformation de la chambre lors de l'écoulement des fluides et facilite sa manipulation. Le support peut être sous forme d'un cadre.

Selon la figure 1 qui représente une vue en éclatée de la chambre, la chambre 1 est formée d'une superposition de plusieurs films. Les films extrêmes 2,3 forment les parois supérieure et inférieure de la chambre et les films intermédiaires 4a,4b,4c,5a,5b sont sous forme de cadre et définissent la zone utile de la chambre.

Les termes "supérieur" et "inférieur" sont définis par rapport à la chambre lorsqu'elle est disposée de sorte à ce que l'écoulement des fluides soit horizontal. Dans cette configuration, le terme "épaisseur" est défini dans la dimension verticale.

Ces films sont avantageusement réalisés dans le même matériau que les parois de la chambre. Les films sont assemblés par exemple par soudage ou collage.

L'utilisation d'un ou plusieurs cadres permet de s'assurer de l'homogénéité de l'épaisseur du canal sur la largeur et la longueur de la chambre.

Pour permettre l'introduction de fluide dans la chambre, celle-ci est munie d'au moins une entrée 6b pour le fluide à irradier.

Pour permettre le retrait des fluides, la chambre d'irradiation comprend au moins une sortie 7b pour le fluide irradié.

Selon la figure 1, la chambre dispose de trois entrées 6a,6b,6c et de trois sorties 7a,7b,7c pour les fluides. Le fluide à irradier est acheminé dans la chambre 1 par l'entrée centrale 6b et un fluide neutre du point de vue de l'irradiation est acheminé par les entrées supérieure 6c et inférieure 6a.

Un fluide neutre du point de vue de l'irradiation est un fluide au moins partiellement perméable au rayonnement utilisé pour l'irradiation.

Par exemple, lorsque le rayonnement est un rayonnement aux UVC, le fluide est un fluide tel qu'un tampon phosphate, une solution saline, ou une solution additive et/ou de conservation d'un composant sanguin.

Les films intermédiaires 4 et 5 sous forme de cadre créent un espace entre les parois. Ces cadres intermédiaires sont de deux types : les « splitter » 5a,5b qui séparent deux fluides avant qu'ils se rejoignent ; et les cadres intercalaires 4a,4b,4c dans l'ouverture desquels les entrées et sorties de la chambre débouchent. Avec les splitters 5a,5b, ces cadres intercalaires forment la zone utile de la chambre, dans laquelle les fluides sont superposés.

Avantageusement, les entrées 6 et sorties 7 débouchent à différentes altitudes dans l'épaisseur du canal, l'épaisseur étant dans la dimension verticale.

Les entrées 6 et sorties 7 se présentent sous forme de conduits minces dont l'une des extrémités s'étend à l'extérieur de la chambre et est reliée à une source de fluide et l'autre extrémité débouche dans la partie creuse d'un cadre 4.

Selon la figure 2, les conduits formants les entrées 6 et sorties 7 de la chambre d'irradiation 1 sont connectées à des tubulures souples en matière thermoplastique, elles mêmes reliées à des sources de fluides.

Les sources de fluides sont constituées par des récipients 8, 9, 10, 11 de type poche contenant les fluides. Du côté entrée, l'entrée centrale est en communication fluidique avec une poche 8 contenant le fluide à irradier, par exemple un concentré de globules rouges et les entrées supérieure et inférieure sont en communication fluidique avec une poche commune 9 contenant un fluide neutre du point de vue de l'irradiation.

Du côté sortie, la sortie centrale est en communication fluidique avec une poche 10 destinée à contenir le fluide irradié, et les sorties supérieure et inférieure sont en communication fluidique avec une poche commune 11 destinée à recueillir le fluide neutre qui a été irradié.

En variante non représentée, la chambre est de forme cylindrique, comme par exemple décrite dans le document US 2007/0000814. En particulier; la chambre est constituée d'un cylindre plein interne et d'un cylindre creux externe qui est concentrique au premier cylindre, créant ainsi entre les deux cylindres, un canal d'écoulement annulaire. Un splitter à l'entrée de la chambre et un splitter à la sortie de la chambre, tous deux sous forme d'une paroi cylindrique, sont disposés entre les deux cylindres interne et externe. La chambre est pourvue de deux entrées pour deux fluides, arrangées respectivement entre le splitter d'entrée et le cylindre externe, et entre le splitter d'entrée et le cylindre interne. De façon similaire, la chambre est en outre pourvue de deux sorties pour les fluides, arrangées respectivement entre le splitter de sortie et le cylindre externe et entre le splitter de sortie et le cylindre interne. Une chambre cylindrique à trois entrées et trois sorties est également envisageable.

### Le dispositif de focalisation

Selon l'invention, l'écoulement du fluide à irradier est focalisé dans une portion déterminée de la chambre d'irradiation selon une certaine géométrie.

La focalisation du fluide inséré par l'entrée centrale est notamment réalisée par l'installation des écoulements dans le canal et plus généralement par l'application d'au moins un champ de force apte à déplacer les particules dans une portion déterminée de la chambre, comme la gravitation, la force centrifuge, magnétique, électrique, acoustique, optique, thermique et/ou la portance.

La géométrie de l'écoulement, et notamment ses dimensions telles que l'épaisseur de la couche de fluide à irradier, est agencée pour qu'une dose d'un rayonnement modifie les propriétés physiques, chimiques et/ou biologiques du fluide.

En effet, la focalisation permet notamment d'obtenir un écoulement du fluide à irradier dont la géométrie est une couche présentant une épaisseur réduite par rapport à l'épaisseur de la portion de la chambre d'irradiation dans laquelle le fluide à irradier est focalisé, c'est-à-dire que l'écoulement du fluide à irradier est distant d'au moins une paroi ou une partie d'une paroi de la chambre. Par exemple, la couche de fluide à irradier possède une épaisseur inférieure à 85%, plus particulièrement 50%, de l'épaisseur de la chambre d'irradiation.

Cette épaisseur est suffisamment réduite pour permettre la pénétration d'un rayonnement déterminé sur une certaine épaisseur de la couche de fluide.

Plus particulièrement, pour réaliser la focalisation, on forme dans la chambre d'irradiation une couche de fluide à irradier ayant une épaisseur déterminée et un écoulement laminaire dans au moins une portion de la chambre.

Par exemple, l'épaisseur de la couche de fluide est réduite de sorte à ce qu'un rayonnement UVC émettant à une longueur d'onde d'environ 254 nm pénètre dans la couche, en maintenant une intensité suffisante pour produire l'effet désiré.

En particulier, la focalisation est réalisée par un champ de force hydrodynamique à l'aide d'au moins une couche, par exemple deux couches, d'un fluide neutre du point de vue de l'irradiation. Les différentes couches, à savoir celle du fluide à irradier et celle(s) du fluide neutre, sont laminaires.

Par couches laminaires, on entend des couches qui glissent les unes sur les autres sans apparition de turbulences.

Plus particulièrement, on établit dans la chambre d'irradiation un écoulement dans une première direction du fluide à irradier selon un premier débit et un écoulement dans ladite première direction d'un fluide neutre du point de vue de l'irradiation selon un second débit, de sorte à former deux couches laminaires ayant chacune une épaisseur déterminée dans au moins une portion de la chambre.

Le fluide à irradier et le fluide neutre sont introduits dans la chambre d'irradiation par un même côté. Ils sont introduits simultanément ou non dans la chambre d'irradiation. Cependant, les deux écoulements de fluides doivent être présents ensembles dans la chambre d'irradiation à un moment donné. Lorsqu'ils sont introduits dans la chambre d'irradiation, les fluides se répartissent sur l'épaisseur du canal de la chambre.

En faisant varier les débits du fluide à irradier et du fluide neutre, l'épaisseur des couches des fluides et leurs positions dans l'épaisseur du canal varient en conséquence. Par exemple, pour obtenir une couche mince de fluide à irradier, le débit du fluide à irradier est plus faible que le débit du fluide neutre. En ajustant les débits, une couche de fluide à irradier d'épaisseur inférieure à 100 µm peut être obtenue.

L'épaisseur de la couche du fluide à irradier ne dépend donc pas directement de l'épaisseur du canal de la chambre d'irradiation, mais est construite par manipulation hydrodynamique à l'intérieur de celle-ci, par la superposition du fluide à traiter et de couches d'un fluide neutre.

En particulier, les débits des fluides sont compris entre 0.1 ml/min et 200 ml/min, notamment entre 1 et 50 ml/min.

Pour diminuer le temps de traitement du fluide, plusieurs chambres d'irradiation reliées à une même source de fluide à irradier et une ou plusieurs sources de fluide neutre sont mises en parallèle. Ainsi, même si les débits dans une chambre d'irradiation sont faibles, la pluralité de chambres d'irradiation permet de traiter une quantité de fluide plus importante qu'avec une seule chambre dans un temps acceptable.

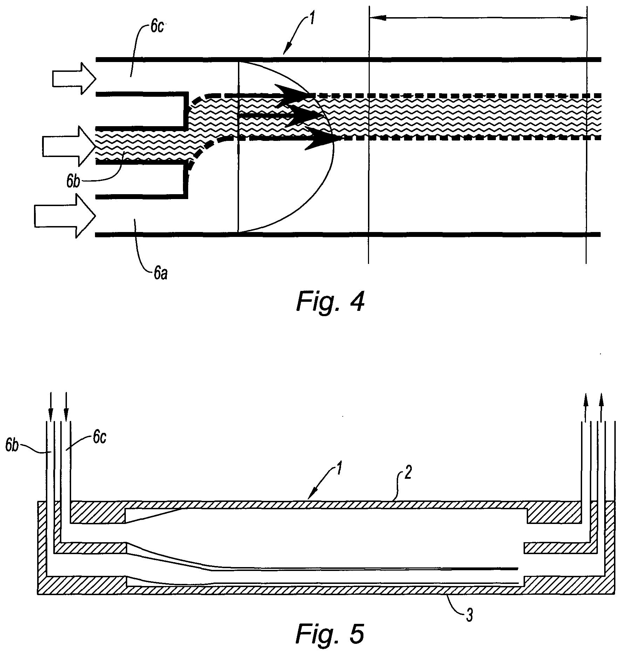

Dans le cas où la chambre ne comprend que deux entrées 6b, 6c pour le fluide à irradier et le fluide neutre, comme illustré sur la figure 5, le fluide à irradier se focalise alors le long de l'une des parois 3 de la chambre 1.

L'utilisation d'une chambre à trois entrées permet d'éviter que le fluide à irradier ne soit en contact avec les parois de la chambre. Par exemple, dans le cas d'un concentré de globules rouges cette caractéristique permet de diminuer le cisaillement appliqué au fluide donc de limiter l'hémolyse. Il est donc avantageux de focaliser la couche de fluide à irradier entre deux couches de fluide neutre.

Dans ce cas, on établit dans la chambre d'irradiation un écoulement dans la même direction que celle de l'écoulement du premier fluide, d'un deuxième fluide neutre du point de vue de l'irradiation, de sorte à former trois couches laminaires, la couche du fluide à irradier étant disposée entre les deux autres couches de fluides neutres.

Les fluides neutres sont identiques ou différents. Selon la figure 1, les fluides neutres sont introduits par les entrées 6a, 6c extrêmes de la chambre d'irradiation, et le fluide à irradier est introduit par l'entrée centrale 6b de la chambre. Ainsi, le fluide à irradier est mis en sandwich entre les deux couches de fluides neutres, celles-ci exerçant un effet protecteur vis-à-vis notamment des parois de la chambre, sur le fluide à irradier.

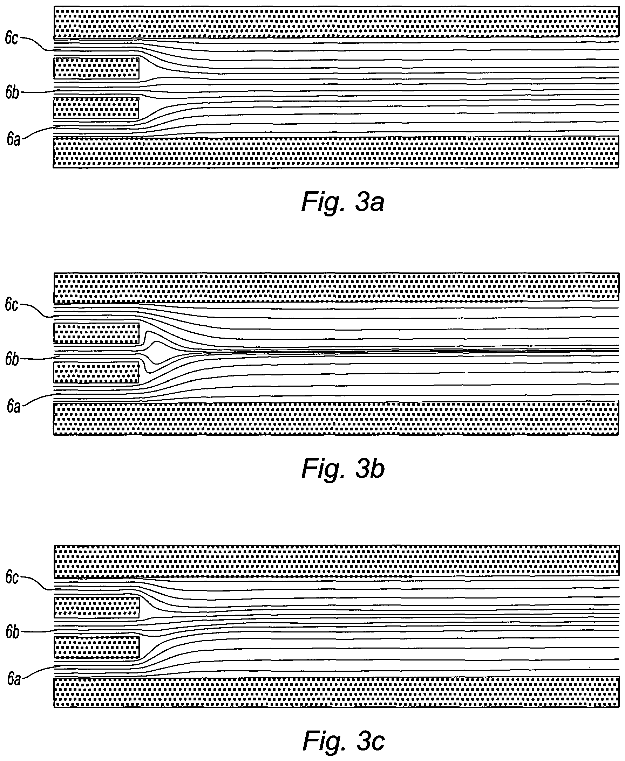

Les figures 3a à 3c illustrent trois modes de mise en oeuvre de la focalisation du fluide avec une chambre à trois entrées et trois sorties. Sur la figure 3a, les débits du fluide à irradier provenant de l'entrée centrale 6b et des fluides neutres provenant des entrées extrêmes 6a et 6c sont égaux. L'écoulement du fluide à irradier se focalise au centre de la chambre 1.

Sur la figure 3b, les débits des fluides neutres provenant des entrées supérieure 6c et inférieure 6a de la chambre sont identiques et plus élevés que le débit du fluide à irradier pénétrant par l'entrée centrale 6b de la chambre. Le fluide à irradier se focalise au centre de l'épaisseur de la chambre, avec une épaisseur réduite par rapport à l'épaisseur de l'écoulement de la figure 3a.

Sur la figure 3c, le débit du fluide neutre provenant de l'entrée inférieure 6a est plus élevé que le débit du fluide neutre provenant de l'entrée supérieure 6c. La couche de fluide à irradier est alors déplacée vers le haut de la chambre.

Selon une réalisation, l'amenée des écoulements des fluides est réalisée à l'aide d'un dispositif de circulation de fluide destiné à établir les écoulements de fluide dans la chambre d'irradiation, comprenant un moyen de mise en circulation de fluide, tel qu'une pompe, par exemple une pompe péristaltique, et un moyen de contrôle des débits, par exemple un régulateur de pompe.

Selon la figure 2, le dispositif de circulation de fluide comprend un ensemble de cinq pompes 12a à 12e.

Une première pompe 12a et une deuxième pompe 12b sont disposées sur une des tubulures connectées à la source de fluide neutre 9, respectivement. Ces deux pompes entraînent le fluide neutre dans la chambre d'irradiation.

Une troisième pompe 12c et une quatrième pompe 12d sont disposées sur chacune des tubulures connectées à la poche 11 destinée à recueillir le fluide neutre irradié. La cinquième pompe 12e est disposée sur la tubulure reliée à la poche 10 destinée à recueillir le fluide irradié.

On note que dans cette mise en oeuvre, il n'est pas nécessaire de pomper le fluide à irradier dans la chambre. En effet, la chambre est rigide et l'équilibre hydrostatique de pression aspire le fluide à irradier lorsque les débits imposés sont tels que Qa+Qc+Qd+Qe+Qf<0, avec :
- Qa le débit de l'entrée supérieure de fluide
- Qb le débit de l'entrée centrale de fluide
- Qc le débit de l'entrée inférieure de fluide
- Qd le débit de la sortie supérieure de fluide
- Qe le débit de la sortie centrale de fluide
- Qf le débit de la sortie inférieure de fluide
Les signes de débits sont tels que le signe positif indique que l'on pousse un fluide dans la chambre et le signe négatif indique que l'on aspire un fluide de la chambre.

Le contrôle et la régulation des écoulements des fluides dans la chambre d'irradiation permettent de focaliser le fluide à irradier dans une portion déterminée de la chambre, sur une épaisseur déterminée et à une vitesse déterminée.

Par exemple, dans le cas d'une chambre à trois entrées, ceci est mis en évidence par la relation : Qa/Q = 3 Wa² - 2 Wa³, avec Wa le rapport d'épaisseur de la couche de fluide issue de l'entrée supérieure, c'est à dire son épaisseur divisée par l'épaisseur de la chambre et Q=Qa+Qb+Qc=-Qd-Qe-Qf. Cette relation peut être aisément démontrée avec les hypothèses suivantes :
- la chambre est beaucoup plus large qu'elle n'est épaisse, le profil de vitesse est donc parabolique selon l'épaisseur,
- les fluides sont uniformes et newtoniens.

On constate donc que l'épaisseur de la couche de fluide ne dépend que du ratio de débits d'entrée. De même, on a Qc/Q = 3 Wc² - 2Wc³ pour l'entrée inférieure. Par contre, l'épaisseur de la couche issue de l'entrée centrale suit une autre équation, car elle n'a pas de contact avec une paroi. On a Qb/Q = 1-3Wa²-3Wc³+2Wa²+2Wc², ce qui peut être vérifié par Qb/Q = 1-Qa/Q-Qc/Q. Ainsi, il est aisé, en partant des épaisseurs souhaitées, de calculer les débits nécessaires. Ces épaisseurs sont fixées en fonction du fluide à irradier.

### Le dispositif d'irradiation

Selon l'invention, après la formation de la couche mince de fluide à irradier, on applique une dose de rayonnement au fluide. La dose est déterminée sur la base de la vitesse du fluide dans la chambre, de l'épaisseur de ladite couche de fluide et de la taille de fenêtre d'irradiation. Cette dose est suffisante pour provoquer l'effet désiré sur le fluide à traiter et notamment pour modifier les propriétés physiques, chimiques et/ou biologiques du fluide à irradier.

Les propriétés physiques, chimiques et/ou biologiques sont par exemple la perte ou la réduction de l'activité biologique, telle que le pouvoir de réplication, des pathogènes éventuellement présents dans le fluide, ou la perte d'immunogénicité des plaquettes ou des globules rouges.

En effet, dans les traitements par irradiation, la dose de rayonnement reçue par le fluide détermine l'efficacité du traitement. Dans les procédés d'inactivation des pathogènes, cette dose doit être suffisante pour éliminer les pathogènes. Elle doit cependant ne pas être trop importante pour ne pas endommager les propriétés biologiques du fluide à traiter, le cas échéant.
La dose reçue par le fluide dépend notamment de la vitesse du fluide dans la chambre d'irradiation et de l'épaisseur du fluide. D'autres paramètres à prendre en compte sont la perméabilité au rayonnement de la chambre d'irradiation, du ou des fluides neutres et du fluide à irradier et la puissance de la source de rayonnement.
Dans une variante, le procédé de l'invention comprend la détermination de la vitesse et de l'épaisseur de l'écoulement du fluide à irradier dans la portion de la chambre dans laquelle le fluide est focalisé. Cette détermination est réalisée de façon avantageuse préalablement à l'irradiation.
En variante, on détermine en continu l'opacité du fluide à irradier dans la ou les gammes de fréquence de l'irradiation à effectuer.

Cette ou ces étapes permettent de contrôler et éventuellement d'ajuster la dose de rayonnement à appliquer à l'écoulement de fluide et/ou la géométrie dudit écoulement focalisé.
La dose de rayonnement est appliquée à l'aide d'un dispositif d'irradiation comprenant un ou des moyens de génération de rayonnement. Le dispositif d'irradiation est apte à délivrer une dose de rayonnement déterminée en fonction de la vitesse et de l'épaisseur de la couche de fluide à irradier et suffisante pour modifier les propriétés souhaitées du fluide à irradier.

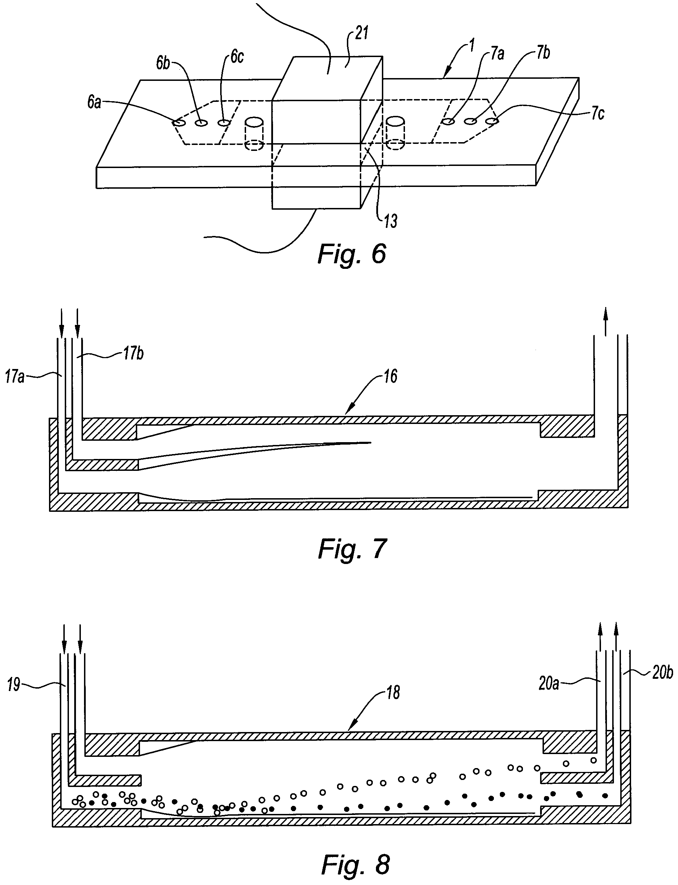

Par exemple et selon la figure 6, le moyen de génération de rayonnement 21 est constitué par une source lumineuse telle qu'une lampe fluorescente, des diodes électroluminescentes, des lampes à décharges ou des lampes à filament. Le moyen de génération de rayonnement peut également comprendre d'autres éléments tels qu'un réflecteur, un filtre ou une lentille optique.

Le moyen de génération du rayonnement est disposé au dessus, en dessous ou de part et d'autre de la chambre d'irradiation de sorte à irradier transversalement l'écoulement de fluide à irradier.

Notamment et en relation avec la figure 2, une portion déterminée 13 de la zone utile de la chambre est dédiée au passage du rayonnement à appliquer au fluide. Des portions peuvent également servir à placer un ou plusieurs capteurs 14a,14b sans contact de manière à effectuer différentes mesures en amont et en aval de la zone à irradier. En particulier, on veut dans certaines implémentations pouvoir mesurer l'épaisseur, la vitesse, et/ou l'opacité de la couche de fluide à irradier.

Ces capteurs par exemple permettent alors de contrôler le bon fonctionnement du dispositif de mise en circulation des fluides et/ou du dispositif d'irradiation.

Selon une réalisation particulière, le procédé comprend l'étape préalable à l'irradiation, de réguler les débits des fluides en fonction de l'épaisseur voulue de fluide à irradier et de l'épaisseur constatée.

De même, le procédé peut comprendre l'étape supplémentaire de régulation de l'intensité du rayonnement en fonction de l'épaisseur constatée de fluide à irradier.

Dans le canal de la chambre d'irradiation, le fluide à irradier possède un profil de vitesse parabolique, comme illustré sur la figure 4. Ainsi dans le cas d'un concentré de cellules, les cellules au centre de la couche de fluide auront une vitesse légèrement supérieure à celle des cellules placées aux extrémités de la couche de fluide.

Ce profil particulier permet de s'assurer, sous certaines conditions, que chaque cellule est irradiée puisque, même si à un moment donné, deux cellules sont dans le même axe d'irradiation, l'instant d'après, elles seront décalées puisqu'elles n'ont pas exactement la même vitesse dans le canal. Pour cela, il faut que le fluide soit strictement dans une moitié ou l'autre de l'épaisseur de la chambre, ou que l'irradiation se fasse par le dessus et le dessous de la chambre.

Selon un deuxième aspect, l'invention concerne un système pour la mise en oeuvre d'un procédé pour modifier les propriétés physiques, chimiques et/ou biologiques d'un fluide par irradiation avec une dose d'un rayonnement, comprenant :
- une chambre d'irradiation 1 comportant au moins une entrée 6b pour l'introduction du fluide à irradier dans la chambre d'irradiation, et une sortie 7b pour le retrait du fluide irradié,
- un dispositif d'irradiation du fluide qui est apte à délivrer une dose de rayonnement ;
- un dispositif de focalisation d'un écoulement du fluide dans une portion déterminée de la chambre d'irradiation 1, selon une géométrie agencée pour que la dose modifie les propriétés physiques, chimiques et/ou biologiques du fluide à irradier.

Selon un aspect particulier, le dispositif de focalisation de l'écoulement comprend un dispositif de circulatiuon 12a, 12b, 12c, 12d, 12e de fluide destiné à établir les écoulements de fluide dans la chambre d'irradiation.

Avantageusement, le système comprend en outre au moins un capteur apte à déterminer la vitesse et l'épaisseur de la couche du fluide à irradier dans la portion de la chambre.

En outre, le système comprend au moins un capteur permettant de déterminer en continu l'opacité du fluide à irradier dans la ou les gammes de fréquence de l'irradiation à effectuer.

En particulier, le système comprend en outre un dispositif de contrôle comprenant un moyen d'asservissement apte à réguler les débits en fonction de l'épaisseur voulue de fluide à irradier et de l'épaisseur constatée et/ou un moyen d'asservissement apte à réguler l'intensité du rayonnement en fonction de l'épaisseur constatée du fluide à irradier.

Sur la figure 2, le dispositif de contrôle est par exemple un microcontrôleur 15.

### Le système à plusieurs modules (mélange et tri)

Selon une réalisation particulière, la chambre d'irradiation est disposée en série avec d'autres chambres de traitement similaires à celles décrites pour la chambre d'irradiation. Notamment, les chambres de traitement permettent un écoulement laminaire des fluides.

Par exemple, lorsque le fluide à irradier est un composant sanguin additionné d'une substance photosensible, une première chambre destinée à mélanger proportionnellement la substance photosensible et le composant sanguin est disposée en amont de la chambre d'irradiation. Le mélange est réalisé en faisant varier les débits du composant sanguin et d'une solution contenant la substance photosensible.

En particulier, selon la figure 7, la chambre 16 destinée au mélange est pourvue d'au moins deux entrées 17a,17b pour chacun des fluides à mélanger, respectivement. Par exemple, le premier fluide est le sang ou le composant sanguin, et le deuxième fluide est une solution contenant une substance photosensible telle qu'une phénothiazine. Par application d'un champ de force transversal à l'écoulement des fluides, et en ajustant les débits des deux fluides à mélanger dans la chambre de mélange, le ratio de débit entre les deux fluides est déterminé. Ce ratio est notamment également fonction de la géométrie de la chambre, des coefficients de diffusion des fluides et du dosage souhaité.

Dans un autre exemple, la chambre en amont de la chambre d'irradiation est une chambre destinée à la séparation de fluide, de sorte à séparer le fluide à irradier. Notamment, la chambre à séparer permet de séparer les différents types de cellule du sang (C.Bor Fuh et J.C.Giddings, 1995).

Dans un autre exemple, en aval de la chambre d'irradiation, on peut disposer une deuxième chambre de séparation destinée à séparer les cellules traitées par illumination des pathogènes et/ou des pathogènes résiduels.

Dans un autre exemple, l'irradiation modifie entre autres les propriétés des particules par rapport à un champ de force donné. La chambre de séparation en aval tire alors profit de cette modification de caractéristique pour séparer les particules illuminées.

Selon la figure 8, une chambre 18 destinée à la séparation comprend au moins une entrée 19 pour le fluide composite à séparer et deux sorties 20a; 20b pour les éléments séparés. En appliquant un champ de force perpendiculaire au plan de l'écoulement des fluides, les particules comprises dans le fluide composite vont migrer selon leur taille et leur coefficient de diffusion, de sorte à les séparer.

## Revendications

1. Procédé pour modifier les propriétés physiques, chimiques et/ou biologiques d'un fluide par irradiation avec une dose d'un rayonnement, ledit procédé comprenant :
- l'amenée d'un écoulement du fluide à irradier dans une chambre d'irradiation, et
- l'application de ladite dose de rayonnement sur l'écoulement du fluide à irradier dans une portion déterminée de la chambre d'irradiation, ledit écoulement étant focalisé dans ladite portion selon une géométrie qui est agencée pour que ladite dose modifie les propriétés dudit fluide, ledit procédé étant **caractérisé en ce que** ladite géométrie est une couche laminaire présentant une épaisseur réduite par rapport à celle de la chambre d'irradiation dans ladite portion.

2. Procédé selon la revendication 1, **caractérisé en ce que** la focalisation est réalisée par l'application sur le fluide d'au moins une force, gravitationnelle, centrifuge, acoustique, magnétique, optique, électrique, thermique et/ou de portance.

3. Procédé selon la revendication 1, **caractérisé en ce que** la focalisation est réalisée par la formation dans ladite portion de la chambre d'une couche du fluide à irradier et d'au moins une couche d'un fluide neutre d'un point de vue de l'irradiation, lesdites couches étant laminaires.

4. Procédé selon la revendication 3, **caractérisé en ce que** la focalisation est réalisée par la formation dans la portion de la chambre de trois couches laminaires, la couche du fluide à irradier étant disposée entre deux couches de fluide neutre du point de vue de l'irradiation.

5. Procédé selon l'une quelconque des revendications 1 à 4, **caractérisé en ce que** la chambre d'irradiation comprend un canal d'épaisseur micrométrique ou millimétrique dans lequel la portion de ladite chambre est formée.

6. Procédé selon l'une quelconque des revendications 1 à 5, **caractérisé en ce qu'**il comprend la détermination de l'opacité dans la ou les gammes de fréquence du rayonnement, de la vitesse et/ou de l'épaisseur de l'écoulement du fluide à irradier, la dose du rayonnement et/ou la géométrie de l'écoulement focalisé étant contrôlée et éventuellement ajustée en fonction de cette détermination.

7. Procédé selon l'une quelconque des revendications 1 à 6, **caractérisé en ce que** le rayonnement est un rayonnement ultraviolet.

8. Procédé selon l'une quelconque des revendications 1 à 7, **caractérisé en ce que** le fluide à irradier est le sang total ou un composant sanguin, éventuellement additionné d'une substance photosensible et/ou d'une solution additive et/ou de conservation.

9. Procédé selon l'une quelconque des revendications 1 à 8, **caractérisé en ce que** l'irradiation est réalisée en vue de réduire les contaminations pathogènes du fluide à irradier.

10. Système pour la mise en oeuvre d'un procédé selon l'une quelconque des revendications 1 à 9 comprenant :
- une chambre d'irradiation (1) comportant au moins une entrée (6b) pour l'introduction du fluide à irradier dans la chambre d'irradiation, et une sortie (7b) pour le retrait du fluide irradié,
- un dispositif d'irradiation (21) du fluide qui est apte à délivrer une dose de rayonnement ;
- un dispositif de focalisation d'un écoulement du fluide dans une portion déterminée de la chambre d'irradiation (1), selon une géométrie agencée pour que la dose modifie les propriétés physiques, chimiques et/ou biologiques du fluide à irradier, **caractérisé en ce que** ladite géométrie est une couche laminaire présentant une épaisseur réduite par rapport à celle de la chambre d'irradiation dans ladite portion.

11. Système selon la revendication 10, **caractérisé en ce qu'**il comprend en outre au moins un capteur (14a,14b) apte à déterminer la vitesse, l'épaisseur ou l'opacité dans la ou les gammes de fréquence dudit rayonnement de la couche du fluide à irradier dans la portion de la chambre.

12. Système selon l'une des revendications 11, **caractérisé en ce qu'**il comprend en outre un dispositif de contrôle (15) comprenant un moyen d'asservissement apte à réguler les débits en fonction de l'épaisseur souhaité de fluide à irradier et de l'épaisseur constatée et/ou un moyen d'asservissement apte à réguler l'intensité du rayonnement en fonction de l'épaisseur constatée de fluide à irradier.

13. Système selon l'une quelconque des revendications 10 à 12, **caractérisé en ce qu'**il comprend, en amont de la chambre d'irradiation, une chambre (16) destinée à la séparation de fluides pour obtenir le fluide séparé ou destinée au mélange du fluide à irradier.

14. Système selon l'une quelconque des revendications 10 à 13, **caractérisé en ce qu'**il comprend, en aval de la chambre d'irradiation, une chambre (18) destinée à la séparation du fluide irradié.

## Patentansprüche

1. Verfahren zum Ändern der physikalischen, chemischen und/oder biologischen Eigenschaften eines Fluids durch Bestrahlung mit einer Dosis einer Strahlung, wobei das Verfahren umfasst:
- Zuführen einer Strömung des zu bestrahlenden Fluids in eine Bestrahlungskammer, und
- Anwenden der Strahlungsdosis auf die Strömung des zu bestrahlenden Fluids in einem bestimmten Abschnitt der Bestrahlungskammer,
wobei die Strömung in dem Abschnitt entsprechend einer Geometrie fokussiert ist, die angeordnet ist, damit die Dosis die Eigenschaften des Fluids ändert, wobei das Verfahren **dadurch gekennzeichnet ist, dass** die Geometrie eine laminare Schicht ist, die eine Stärke aufweist, die im Verhältnis zu jener der Bestrahlungskammer in dem Abschnitt reduziert ist.

2. Verfahren nach Anspruch 1, **dadurch gekennzeichnet, dass** die Fokussierung durch Anwenden auf das Fluid von mindestens einer Schwerkraft, einer Zentrifugalkraft, einer akustischen, magnetischen, optischen, elektrischen, thermischen und/oder Auftriebskraft durchgeführt wird.

3. Verfahren nach Anspruch 1, **dadurch gekennzeichnet, dass** die Fokussierung durch das Bilden einer Schicht des zu bestrahlenden Fluids und mindestens einer Schicht eines in Bezug auf die Bestrahlung neutralen Fluids in dem Abschnitt der Kammer durchgeführt wird, wobei die Schichten laminar sind.

4. Verfahren nach Anspruch 3, **dadurch gekennzeichnet, dass** die Fokussierung durch die Bildung von drei laminaren Schichten in dem Abschnitt der Kammer durchgeführt wird, wobei die Schicht des zu bestrahlenden Fluids zwischen zwei Schichten eines in Bezug auf die Bestrahlung neutralen Fluids angeordnet wird.

5. Verfahren nach einem der Ansprüche 1 bis 4, **dadurch gekennzeichnet, dass** die Bestrahlungskammer einen Kanal mit einer mikrometrischen oder millimetrischen Stärke umfasst, in dem der Abschnitt der Kammer gebildet wird.

6. Verfahren nach einem der Ansprüche 1 bis 5, **dadurch gekennzeichnet, dass** es die Bestimmung der Opazität der Frequenzspanne(n) der Strahlung, der Geschwindigkeit und/oder der Stärke der Strömung des zu bestrahlenden Fluids umfasst, wobei die Dosis der Strahlung und/oder die Geometrie der fokussierten Strömung in Abhängigkeit von dieser Bestimmung kontrolliert und eventuell angepasst wird.

7. Verfahren nach einem der Ansprüche 1 bis 6, **dadurch gekennzeichnet, dass** die Strahlung eine Ultraviolett-Strahlung ist.

8. Verfahren nach einem der Ansprüche 1 bis 7, **dadurch gekennzeichnet, dass** das zu bestrahlende Fluid das gesamte Blut oder ein Blutbestandteil, eventuell unter Beigabe einer lichtempfindlichen Substanz und/oder einer Additivlösung und/oder Konservierungslösung ist.

9. Verfahren nach einem der Ansprüche 1 bis 8, **dadurch gekennzeichnet, dass** die Bestrahlung in Hinblick auf die Reduzierung der pathogenen Kontaminationen des zu bestrahlenden Fluids durchgeführt wird.

10. System zum Umsetzen eines Verfahrens nach einem der Ansprüche 1 bis 9, umfassend:
- eine Bestrahlungskammer (1), mindestens einen Eingang (6b) zum Zuführen einer Strömung des zu bestrahlenden Fluids in eine Bestrahlungskammer enthaltend, und einen Ausgang (7b) zum Abziehen des bestrahlten Fluids,
- eine Bestrahlungsvorrichtung (21) des Fluids, die imstande ist, eine Strahlungsdosis abzugeben;
- eine Fokussierungsvorrichtung einer Strömung des Fluids in einem bestimmten Abschnitt der Bestrahlungskammer (1) entsprechend einer Geometrie, die angeordnet ist, damit die Dosis die physikalischen, chemischen und/oder biologischen Eigenschaften des zu bestrahlenden Fluids ändert, **dadurch gekennzeichnet, dass** die Geometrie eine laminare Schicht ist, die eine Stärke aufweist, die im Verhältnis zu jener der Bestrahlungskammer in dem Abschnitt reduziert ist.

11. System nach Anspruch 10, **dadurch gekennzeichnet, dass** es weiter mindestens einen Sensor (14a, 14b) umfasst, der imstande ist, die Geschwindigkeit, die Stärke oder die Opazität der Frequenzspanne(n) der Strahlung des zu bestrahlenden Fluids in dem Abschnitt der Kammer zu bestimmen.

12. System nach Anspruch 11, **dadurch gekennzeichnet, dass** es weiter eine Steuervorrichtung (15) umfasst, die ein Ansteuerungsmittel umfasst, das imstande ist, die Flussmengen in Abhängigkeit von der gewünschten zu bestrahlenden Fluidstärke und der festgestellten Stärke zu regeln und/oder ein Ansteuerungsmittel, das imstande ist, die Intensität der Strahlung in Abhängigkeit von der festgestellten Stärke des zu bestrahlenden Fluids zu regeln.

13. System nach einem der Ansprüche 10 bis 12, **dadurch gekennzeichnet, dass** es stromaufwärts der Bestrahlungskammer eine Kammer (16) umfasst, die zur Trennung von Fluiden bestimmt ist, um das getrennte Fluid zu erhalten, oder zum Mischen des zu bestrahlenden Fluids bestimmt ist.

14. System nach einem der Ansprüche 10 bis 13, **dadurch gekennzeichnet, dass** es stromabwärts der Bestrahlungskammer eine Kammer (18) umfasst, die zur Trennung des bestrahlten Fluids bestimmt ist.

## Claims

1. Method of modifying the physical, chemical and/or biological properties of a fluid by irradiation with a radiation dose, said method comprising:
- bringing a fluid flow to be irradiated into an irradiation chamber, and
- application of said radiation dose onto the fluid flow to be irradiated in a determined portion of the irradiation chamber,
said flow being focussed in said portion with a geometry arranged such that said dose modifies the properties of said fluid, said method being **characterised in that** said geometry is a laminar layer thinner than the irradiation chamber in said portion.

2. Method according to claim 1, **characterised in that** focussing is done by application of at least one gravitational, centrifugal, acoustic, magnetic, optical, electrical, thermal and/or lift force on the fluid.

3. Method according to claim 1, **characterised in that** focussing is done by formation of a layer of the fluid to be irradiated and at least one layer of a fluid neutral to irradiation, in said portion of the chamber, said layers being laminar.

4. Method according to claim 3, **characterised in that** focussing is done by formation of three laminar layers in the portion of the chamber, the layer of fluid to be irradiated being located between two layers neutral to irradiation.

5. Method according to any one of claims 1 to 4, **characterised in that** the irradiation chamber comprises a channel with a thickness measured in micrometres or millimetres in which the portion of said chamber is formed.

6. Method according to any one of claims 1 to 5, **characterised in that** it includes determination of the opacity in the radiation frequency range(s), the velocity and/or thickness of the fluid flow to be treated, the radiation does and/or the focussed flow geometry being controlled and possibly adjusted as a function of this determination.

7. Method according to any one of claims 1 to 6, **characterised in that** the radiation is an ultraviolet radiation.

8. Method according to any one of claims 1 to 7, **characterised in that** the fluid to be irradiated is total blood or a blood component, possibly to which a photosensitive substance and/or an additive and/or preservative solution may be added.

9. Method according to any one of claims 1 to 8, **characterised in that** irradiation is done to reduce pathogenic contaminations of the fluid to be irradiated.

10. System for use of a method according to any one of claims 1 to 9 comprising:
- an irradiation chamber (1) comprising at least one inlet (6b) through which fluid to be irradiated is brought into the irradiation chamber, and an outlet (7b) through which the irradiated fluid is extracted,
- a fluid irradiation device (21) that can output a radiation dose;
- a device to focus a fluid flow in a determined portion of the irradiation chamber (1) according to a geometry arranged such that the dose modifies the physical, chemical and/or biological properties of the fluid to be irradiated, **characterised in that** said geometry us a laminar layer thinner than the irradiation chamber in this portion.

11. System according to claim 10, **characterised in that** it comprises at least one sensor (14a, 14b) capable of determining the velocity, thickness or opacity of the fluid layer to be irradiated in the portion of the chamber, in the frequency range(s) of said radiation.

12. System according to claim 11, **characterised in that** it also comprises a control device (15) comprising a slaving means capable of regulating flows as a function of the required thickness of the fluid to be irradiated and the observed thickness and/or a slaving means capable or regulating the radiation intensity as a function of the observed thickness of the fluid to be irradiated.

13. System according to any one of claims 10 to 12, **characterised in that** it also comprises a chamber (16) on the input side of the irradiation chamber in which fluids will be separated to obtain the separated fluid or the fluid to be irradiated will be mixed.

14. System according to one of claims 10 to 13, **characterised in that** it comprises a chamber (18) on the output side of the irradiation chamber, in which the irradiated fluid will be separated.
